Europäisches Patentamt

European Patent Office

Office européen des brevets

(11) Publication number: **0 352 034**
**A2**

# EUROPEAN PATENT APPLICATION

(21) Application number: **89307160.5**

(22) Date of filing: **14.07.89**

(51) Int. Cl.4: **C07C 43/12 , C07C 309/73 , C07C 41/22**

(30) Priority: **18.07.88 US 220716**

(43) Date of publication of application:
**24.01.90 Bulletin 90/04**

(84) Designated Contracting States:
**DE FR GB IT**

(71) Applicant: **BOC, INC.**
**575 Mountain Avenue**
**Murray Hill New Jersey 07974(US)**

(72) Inventor: **Robin, Mark L.**
**245 East Golf Avenue**
**South Plainfield New Jersey 07080(US)**
Inventor: **Halpern, Donald F.**
**156 Marian Avenue**
**Fanwood New Jersey 07023(US)**

(74) Representative: **Wickham, Michael et al**
**c/o Patent and Trademark Department The**
**BOC Group plc Chertsey Road**
**Windlesham Surrey GU20 6HJ(GB)**

(54) Process and intermediate compound for the preparation of CHF2OCHFCF3.

(57) 2-(difluoromethoxy)-1,2,2,2-tetrafluoroethane ($CHF_2OCHFCF_3$), a known inhalation anaesthetic, is prepared by a synthetic route in which a first and novel intermediate:

$p\text{-}CH_3C_6H_4CHF_2OCHFCF_3$

is prepared by reacting methyl hemiacetal ($CF_3CH(OH)OCH_3$) with para-toluene sulphonyl chloride in the presence of an organic base such as triethylamine at room temperature. The resulting novel tosylate intermediate is then converted to a second intermediate $CF_3CHFOCH_3$ by reaction at elevated temperature with a fluorinating agent such as caesium fluoride. The second intermediate is converted by known means to $CHF_2OCHFCF_3$ (eg by photochemical chlorination) followed by fluorination of the resulting compound with HF in the presence of $SbCl_5$.

## PROCESS AND INTERMEDIATE COMPOUND FOR THE PREPARATION OF THE CH$_2$OCHFCF$_3$

This invention relates to the preparation of 2-(difluoromethoxy)-1,2,2,2-tetrafluoroethane (CHF$_2$OCHFCF$_3$), an important volatile liquid inhalation anaesthestic particularly suited for administration to patients during outpatient surgery and to the preparation of intermediate compounds in its synthesis.

A common method of preparing CHF$_2$OCHClCF$_3$ is by reacting isoflurane (CHF$_2$OCHClCF$_3$) with a fluorinating agent such as BrF$_3$. Isoflurane, however, is expensive and significantly increases the cost of producing CHF$_2$OCHFCF$_3$.

Isoflurane's expense is caused mainly by its method of preparation from trifluoroethanol (CF$_3$CH$_2$OH) and chlorodifluoromethane (CF$_2$HCl). Trifluoroethanol is both difficult to produce and expensive to obtain.

In view of the above, the compound fluoral methyl hemiacetal (CF$_3$CH(OH)OCH$_3$) is an attractive alternative starting material for the preparation of CHF$_2$OCHFCF$_3$. CF$_3$CH(OH)OCH$_3$ can be economically produced in high yield from the reaction of fluoral (CF$_3$CHO) with methanol, both of which are inexpensive materials.

Fluoral methyl hemiacetal has indeed been used as a starting material for the production of CHF$_2$OCHFCF$_3$. For example, US Patent No. 3,980,714 discloses reacting the hemiacetal with PCl$_5$ or SOCl$_2$ thereby to replace the hydroxy group with a chlorine atom This product (CF$_3$CHClOCH$_3$) is photochemically chlorinated to produce the compound CF$_3$CHClOCHCl$_2$, which is then reacted with a fluorinating agent to produce the desired product (CHF$_2$OCHFCF$_3$), as disclosed in German Offen. 2,823,969.

In a process disclosed in US Patent No. 3,981,927, the hemiacetal is reacted with a compound of the formula R′R′NCF$_2$CHFCl to replace the hydroxy group with a fluorine atom. The fluorinated product is then reacted with chlorine to produce a compound of the formula CF$_3$CHFOCHCl$_2$ which in turn is reacted with a fluorinating agent such as hydrogen fluoride to produce the desired product CHF$_2$OCHFCF$_3$. This last reaction is disclosed in US Patent 3535 388.

The above procedures are disadvantageous because they employ complex and costly reagents.

The invention relates to an alternative process for preparing CHF$_2$OCHFCF$_3$ which employs a novel intermediate compound of formula: p-CH$_3$C$_6$H$_4$SO$_2$OCH(OCH$_3$)CF$_3$. This intermediate may be prepared from para-toluene sulphonyl chloride and the aforesaid fluoral methyl hemiacetal, and thus an improved method of making the end compound is made possible.

According to the present invention there is provided a process for the preparation of a compound having the formula CF$_3$CHFOCH$_3$ comprising fluorinating a compound of the formula p-CH$_3$C$_6$H$_4$SO$_2$OCH(OCH$_3$)CF$_3$ to form the said compound. The resulting compound may then be reacted with chlorine gas to effect chlorine substitution on the terminal carbon. The chlorinated compound may then be reacted with a fluorinating agent to obtain high yields of the final product CHF$_2$OCHFCF$_3$.

In a preliminary step of the process, CF$_3$CH(OH)OCH$_3$ may be reacted with p-toluene sulfonyl chloride in the presence of a suitable solvent at ambient or room temperature to produce a novel compound of the formula p-CH$_3$C$_6$H$_4$SO$_2$OCH(OCH$_3$)CF$_3$. The preferred reaction temperature is in the range of about 11 to 30°C, most preferably 20 to 23°C. The preferred solvent is an organic solvent such as dioxane, and the reaction is preferably carried out in the presence of an organic base, for example, triethylamine. When the reaction is conducted in the presence of triethylamine, the molar ratios of the reactants CF$_3$CH(OH)OCH$_3$ to p-toluene sulfonyl chloride to triethylamine can range from about 1 : 1 : 1 to 1 : 3 : 3, preferably being about 1 : to 1.2 : 1.2.

The tosylate reaction product can be separated from the reaction mixture by any convenient procedure, for example, by aqueous work-up of the reaction mass following the removal of precipitated crystals of triethylamine hydrogen chloride. The tosylate compound is then reacted (in accordance with the invention) with a fluorinating agent in a suitable solvent to replace the tosylate group with a fluorine atom thereby to produce a product of the formula CF$_3$CHFOCH$_3$. This reaction is typically carried out at a temperature of about 130 to 250°C, preferably in the range of 160-200°C when using an organic solvent such as for example, diethylene glycol, or at about 100°C when using water as a solvent. Suitable fluorinating agents include inorganic fluorinating agents, particularly fluorides such as potassium fluoride and caesium fluoride.

CF$_3$CHFOCH$_3$ prepared as described above can then be used to prepare CF$_3$CHFOCHF$_2$ by any process known in the art, such as that disclosed in DE-A-2,361,058. In such a process CF$_3$CHFOCHF$_2$ can be conveniently prepared by the photochemical chlorination of 1,2,2,2-tetrafluoroethyl methyl ether (CF$_3$CHFOCH$_3$) followed by fluorination with HF in the presence of SbCl$_5$. Alternatively, the compound CF$_3$CHFOCH$_3$

can be reacted with chlorine gas to form $CF_3FOCHCl_2$ which is in turn reacted with a fluorinating agent, as described in US-A-3 535 388.

Other characteristics and descriptions of $CF_3CHFOCHF_2$ and anaesthetic compositions containing this compound are disclosed in US-A-4,762,856, incorporated herein by the reference. In accordance with the present invention, the reaction of fluoral methyl hemiacetal with p-toluene sulfonyl chloride strongly favours the formation of the corresponding tosylate compound. As a result, the yield of the described product $CHF_2OCHCF_3$ is enhanced over previously known methods.

The following examples are provided more fully to illustrate the present invention.

Example 1

Preparation of p-$CH_3C_6H_4SO_2OCH(OCH_3)CF_3$ from $CF_3CH(OH)OCH_3$ and p-toluene sulfonyl chloride

A one litre flask equipped with a thermometer and stir bar is charged with 25.0g (0.192 moles) of $CF_3CH(OH)OCH_3$, 32 ml (23 g, 0.230 moles) of triethylamine and 200 ml of 1,4-dioxane. The flask and contents are then cooled in an ice bath to approximately 12°C, and a solution of 44.0 g (0.230 moles) of p-toluene sulfonyl chloride in 200 ml of 1,4-dioxane is added dropwise with stirring. The reaction temperature is maintained at approximately 20-23°C.

Following the addition of the p-toluene sulfonyl chloride, the reaction mixture is stirred at room temperature for two hours. Precipitated triethylamine hydrogen chloride is then removed by filtration and the product is isolated by aqueous work-up to yield 41.0 g (69% yield) of p-$CH_3C_6H_4SO_2OCH(OCH_3)CF_3$ having approximately ninety percent purity. The $^1H$ NMR of the product displays a singlet at 2.5 ppm (aromatic $CH_3$), singlet at 3.6 ppm ($OCH_3$), a quartet (J = 4Hz) at 5.6 ppm ($CF_3CH$), and a characteristic pattern for a para-substituted aromatic ring at 7.0-8.0 ppm.

Example 2

Preparation of $CF_3CHFOCH_3$ from p-$CH_3C_6H_4SO_2OCH(OCH_3)CF_3$

A flask is equipped with a thermometer, stir bar and 4 inch Vigreux column to which is attached a reflux head apparatus wherein the cold finger distillation head is maintained at approximately -10°C through circulation of a chilled water/methanol mixture. The flask is charged with 20 g (0.07 moles) of p-$CH_3C_6H_4SO_2OCH(OCH_3)CF_3$, 30 g (0.20 moles) of caesium fluoride, and 100 ml of diethylene glycol. The reaction mixture is heated to 180°C and a total of 2.9 g of product collected. The $^1H$ NMR shows this material to be 66% $CF_3CHFOCH_3$, at a 33% yield. The $^1H$ NMR of the product containing $CF_3CHFOCH_3$ displays a singlet at 3.6 ppm ($OCH_3$) and a doublet of quartets at 5.2 ppm. The $^{19}F$ NMR displays a singlet at -84 pm (CHF) and a doublet at -146 ppm.

Example 3

Preparation of $CF_3CHFOCH_3$ from p-$CH_3C_6H_4SO_2OCH(OCH_3)CF_3$ in aqueous media

(a) A solution of 100g of potassium fluoride dissolved in 100g of water and 15g of crude p-$CH_3C_6H_4SO_2OCH(OCH_3)CF_3$ are added to a 500ml single neck flask equipped with a Dean-Start trap and set for reflux. The reflux condenser is cooled to -10°C by a circulating water/ethylene glycol solution. The reaction mass is heated to reflux and 1.1g of distillate are collected. Gas chromotography [SP 1000 on 60/80 mesh Carbopak B (TM Supelco) 20ft (6.1m) long and 0.125 inches (3.2mm) in diameter at 190°C and He flow of 32 ml/min] shows the distillate contains 57% of the product, $CF_3CHFOCH_3$.

(b) The procedure of part (a) was repeated utilizing a solution of 6og cesium fluoride in 60cc of waer, other conditions and quantities being the same. There was obtained 1.2g of distillate containing 65% $CF_3CHFOCHF_2$ (GC analysis).

Example

Preparation of $CF_3CHFOCH1_2$ from $CF_3CHFOCH_3$

A total of 305.5g (2.34 moles) of $CF_3CHFOCH_3$ was added to a water-jacketed chlorinator fitted with a thermometer, a "Dry-Ice" cold finger-type condenser and a fitted glass gas dispersion tube. The reaction was carried out at 25°C with gaseous chlorine being bubbled through the solution which was exposed to a source of illumination. The effluent HC1 was colleted in an aqueous scrubber and aliquots were titrated with standard base. The reaction was continued until slightly less than 2.0 moles of HC1 per mole of ether was titrated. The reaction product, $CF_3CHFOCHCl_2$, B.P. 85°C at 150 mm was recovered by distillation.

Example 5

Preparation of $CF_3CHFOCHF_2$ from $CF_3CHFOCHCl_2$

A l-liter 3-necked stainless steel flask was fitted with a copper "Dry-Ice" cold finger condenser, a stainless steel stirring shaft and gland and a copper gas inlet tube. To the flask there was added 466 grams of $CF_3CHFOCHCl_2$ and 1.5g of $SbCl_5$. HF gas was then slowly bubbled through the stirred mixture which was maintained at 0°C. Following the fluorination, a product was recovered and distilled through a 60 x2 cm column packed with glass helices to yield purified material with a boiling point range of 23° - 24°C. N.M.R. identified the compound as $CF_3CHFOCHF_2$.

**Claims**

1. A process for the preparation of a compound having the formula $CF_3CHFOCH_3$ comprising fluorinating a compound of the formula p-$CH_3C_6H_4SO_2OCH(OCH_3)CF_3$ to form the said compound of formula $CF_3CHFOCH_3$.

2. A process according to claim 1, wherein the fluorination reaction is conducted in an organic solvent at a temperature in the range 130 to 250° C.

3. A process according to claim 2, wherein the fluorination reaction is conducted at a temperature in the range of 160 to 200° C.

4. A process according to claim 2 or claim 3 wherein the solvent is diethylene glycol.

5. A process according to claim 1, wherein the fluorination reaction is conducted in water at a temperature of about 100°C.

6. A process according to any one of the preceding claims, in which the fluorinating agent is potassium fluoride or caesium fluoride.

7. A process according to any one of the preceding claims, wherein the compound of formula p-$CH_3C_6H_4SO_2OCH(OCH_3)CF_3$ is formed by reacting p-toluene sulphonyl chloride with a compound of formula $CF_3CH(OH)OCH_3$.

8. A process according to claim 7, in which the reaction between p-toluene sulphonyl chloride and the compound of formula $CF_3CH(OH)OCH_3$ is conducted in a solvent at room temperature in the presence of an organic base.

9. A process according to claim 8, in which the organic base is triethylamine.

10. A process according to claim 9, in which the mole ratio of the compound of formula $CF_3CH(OH)OCH_3$ to p-toluene sulphonyl chloride to triethylamine is in the range of from 1 : 1 : 1 to 1 : 3 : 3.

11. A process according to any one of claims 8 to 11, in which the solvent is dioxane.

12. A process for preparing a compound of the formula $CHF_2OCHFCF_3$, wherein a compound of the formula $CF_3OCHFOCH_3$ is converted to said compound of formula $CHF_2OCHFCF_3$, characterised in that the compound of formula $CF_3CHFOCH_3$ is formed by a process according to any one of the preceding claims.

13. A compound having the formula: p-$CH_3C_6H_4SO_2OCH(OCH_3)CF_3$